# EUROPÄISCHE PATENTSCHRIFT

(11) **EP 1 589 887 B1**
(45) Veröffentlichungstag und Bekanntmachung des Hinweises auf die Patenterteilung: **29.12.2010**
(21) Anmeldenummer: 03779640.6
(22) Anmeldetag: 31.12.2003
(51) Int. Cl.: A61B 17/80

(54) **IMPLANTIERBARE ORTHOPÄDISCHE VORRICHTUNG**
IMPLANTABLE ORTHOPAEDIC DEVICE
DISPOSITIF ORTHOPEDIQUE IMPLANTABLE

(30) Priorität: 03.02.2003 CH 150032003
(43) Veröffentlichungstag der Anmeldung: 02.11.2005
(73) Patentinhaber: Stryker Trauma SA, 2545 Selzach (CH)
(72) Erfinder: GASSER, André, CH-4513 Langendorf (CH); LUTZ, Christian, CH-4500 Solothurn (CH); BAUMGARTNER, Thomas, CH-4552 Derendingen (CH); BERNHARD, Andreas, CH-2554 Meinisberg (CH)
(74) Vertreter: Liebetanz, Michael
(86) Internationale Anmeldenummer: PCT/CH2003/000852
(87) Internationale Veröffentlichungsnummer: WO 2004/069066

(56) Entgegenhaltungen:
- DE-A- 10 226 496
- US-A- 5 474 553
- US-A- 5 607 428
- US-A- 6 030 389
- US-B1- 6 235 033

## Beschreibung

Die Erfindung betrifft eine implantierbare orthopädische Vorrichtung mit einem Lastträgerelement, mit mindestens einem polyaxial orientierbaren Fixationselement, wobei in dem Lastträgerelement mindestens eine Öffnung für den Durchgang von polyaxial orientierbaren Fixationselementen vorgesehen ist, und mit einem Einsatz, der in die besagte Öffnung in einer Aufnahme einsetzbar ist, wobei der Einsatz eine zu der inneren Form der Aufnahme komplementäre äussere Form aufweist, die eine polyaxiale Rotation des Einsatzes in der Aufnahme gestattet, wobei der Einsatz eine zentrale Durchbohrung zur Aufnahme des Körpers des Fixationselementes aufweist und wobei die besagte implantierbare orthopädische Vorrichtung Mittel zum Halten des Einsatzes in der Aufnahme aufweist.

Eine solche Vorrichtung ist beispielsweise aus der US 5,954,722 (Basis für den Oberbegriff des Anspruchs 1) bekannt. In der Platte ist mindestens eine Öffnung vorgesehen, die eine Aufnahme definiert, in die ein einteiliger, mit einem durchgehenden Schlitz versehener Einsatz einsetzbar ist, der ein Innengewinde aufweist, in das ein Gewinde des Fixationselementes eingreifen kann. Die Öffnung der Platte ist hohlkugelig ausgestaltet, so dass sie eine Aufnahme für den eine kugelige Aussenfläche aufweisenden Einsatz bildet. Dadurch ist der Einsatz mit einem in diesen eingeschraubten Fixationselement polyaxial orientierbar. Bei einem Einschrauben des Fixationselementes in den Einsatz spreizt dieses den Einsatz auseinander, der nun in der Aufnahme das Fixationselement in dessen Lage fixiert.

Die US 5,607,426 zeigt eine andere Lösung für die polyaxiale Festlegung eines Fixationselementes. In der Platte ist auch mindestens eine Öffnung vorgesehen, die eine Aufnahme definiert, in die ein zweiteiliger Einsatz einsetzbar ist, dessen äusserer Teil ein Aussengewinde aufweist, das in ein knochenfern vorgesehenes Innengewinde der Platte eingreifen kann. Die Platte verfügt auf ihrer knochennahen Seite über eine konische Verjüngung der Öffnung. Gegen diese konische Verjüngung stützt sich der komplementär gestaltete innere Teil des Einsatzes ab. Der innere Teil des Einsatzes ist mehrfach geschlitzt, hohl und zur Aufnahme eines kugeligen Kopfes des Fixationselementes vorgesehen. Damit ist dieses polyaxial orientierbar. Bei einem Eindrehen des äusseren Teil des Einsatzes drückt dieser auf den inneren Teil des Einsatzes, der infolge seiner Verschiebung in der Richtung senkrecht zur Plattenebene durch die konische Verjüngung den besagten Hohlraum verkleinert und damit den kugeligen Kopf des Fixationselementes in dessen Lage fixiert. Es ist klar, dass die polyaxiale Fixierung des kugeligen Kopfes des Fixationselementes vorab geschehen muss und über den Einsatz eines Werkzeuges in eine in diesem Kopf vorgesehene Nut erfolgen kann.

Ausgehend von diesem Stand der Technik liegt der Erfindung die Aufgabe zugrunde, eine Vorrichtung der eingangs genannten Art so zu verbessern, dass sie für den Chirurgen einfacher, schneller und sicherer handhabbar ist.

Diese Aufgabe wird erfindungsgemäss dadurch gelöst, dass der Einsatz aus zwei Einsatzelementen mit der besagten Durchbohrung besteht, dass das erste Einsatzelement die besagte äussere Form aufweist, die die polyaxiale Rotation desselben in der Aufnahme gestattet, dass das erste Einsatzelement einen zentralen inneren Hohlraum aufweist, in dem das zweite Einsatzelement einsetzbar ist, dass das erste Einsatzelement mindestens einen Schlitz derart aufweist, dass bei einer axialen Verschiebung der beiden Einsatzelemente gegeneinander das erste Einsatzelement in der Platte mindestens teilweise aufspreizbar ist, um Position und Ausrichtung des Fixationselementes in der Vorrichtung polyaxial einstellbar zu blockieren.

Dadurch dass ein zweiteiliges Einsatzelement vorgesehen ist, kann in einfacherer Weise eine polyaxiale Verriegelung sichergestellt werden, ohne dass dies für den Chirurgen einen erhöhten Aufwand im Einsatz solchermassen ausgestalteter Knochenplatten bedeutet.

Weitere vorteilhafte Ausführungsformen sind in den Unteransprüchen gekennzeichnet.

Die Erfindung wird nun beispielhaft an zwei Ausführungsbeispielen unter Bezugnahme auf die Zeichnungen beschrieben. Es zeigen:
- Fig. 1: eine geschnittene Seitenansicht eines ersten Ausfüh- rungsbeispiels der Vorrichtung,
- Fig. 2: eine geschnittene 3D-Ansicht des ersten Einsatzelemen- tes aus der Fig. 1,
- Fig. 3: eine geschnittene 3D-Ansicht des zweiten Einsatzelemen- tes aus der Fig. 1,
- Fig. 4: eine geschnittene Seitenansicht einer ersten abgewan- delten Form des ersten Ausführungsbeispiels der Vor- richtung,
- Fig. 5: eine geschnittene Seitenansicht einer zweiten abgewan- delten Form des ersten Ausführungsbeispiels der Vor- richtung,
- Fig. 6: eine in einer anderen Ebene des ersten Einsatzelementes geschnittene Seitenansicht des ersten Ausführungsbei- spiels nach Fig. 1, und
- Fig. 7: eine geschnittene Seitenansicht eines zweiten Ausfüh- rungsbeispiels der Vorrichtung.

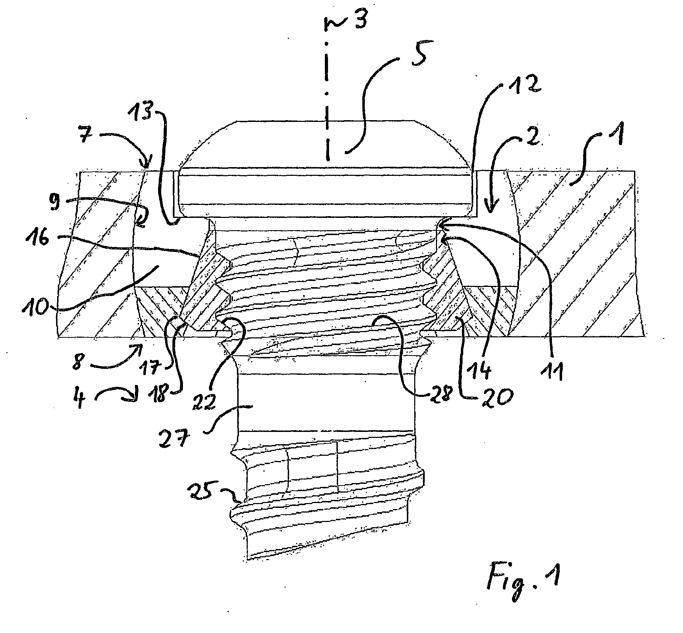

Die Fig. 1 zeigt eine geschnittene Seitenansicht eines ersten Ausführungsbeispiels der Vorrichtung. Mit dem Bezugszeichen 1 ist die Platte bezeichnet, bei der es sich allgemein um eine zur Implantation auf einen Knochen anzulegende vorgesehene Platte 1 handelt, die üblicherweise über eine Vielzahl von quer zu ihrer Hauptebene vorgesehenen Löchern 2 verfügt. Die Hauptachse der Löcher 2 ist mit dem Bezugszeichen 3 bezeichnet, in dem Bereich 4 ist beim Einsatz der Vorrichtung das Knochenmaterial. Statt der Platte 1 kann man auch allgemein von einem Lastträgerelement sprechen, da neben Platten auch andere Lastträger, wie beispielsweise insbesondere Stäbe für den Einsatz der Erfindung vorgesehen werden können.

Die Löcher 2 können (nicht dargestellt) mündungsnah hohlzylindrisch ausgestaltet, so dass sich zu der Hauptachse 3 parallele Aussenwände ergeben. Die Mündungen selber können abgerundet oder sich nach aussen öffnend kegelförmig ausgestaltet sein. Diese parallelen Aussenwände können aber auch anders ausgestaltet sein, zum Beispiel die Öffnung konisch erweiternd oder die Öffnung konisch verkleinernd, um nur zwei einfache rotationssymmetrische Möglichkeiten zu nennen. Wesentlich ist das Vorhandensein von Vorsprüngen 7 bzw. 8. Das bedeutet, dass zwischen diesen Vorsprüngen 7 bzw. 8 eine Aufnahme 9 geschaffen ist, die hohlkugelig ist. Dabei ist der Durchmesser dieser hohlkugeligen Aufnahme 9 grösser als der sich durch die Vorsprünge 7 bzw. 8 ergebende zylinderförmige Lochbereich. Dies gewährleistet, dass flanschartige umlaufende Vorsprünge 7 bzw. 8 bestehen, die ein zumindest in Teilbereichen kugelartiges Einsatzelement 10 sicher in der Aufnahme 9 halten.

Dieses als erstes zu bezeichnende kugelige Einsatzelement 10 kann sich dann frei um seinen Kugelmittelpunkt drehen, der in der Achse 3 liegt, die die in der Fig. 1 dargestellte senkrechte Ausrichtung des ersten Einsatzelementes 10 darstellt. Das erste Einsatzelement 10 kann aber nicht aus der Aufnahme 9 herausfallen.

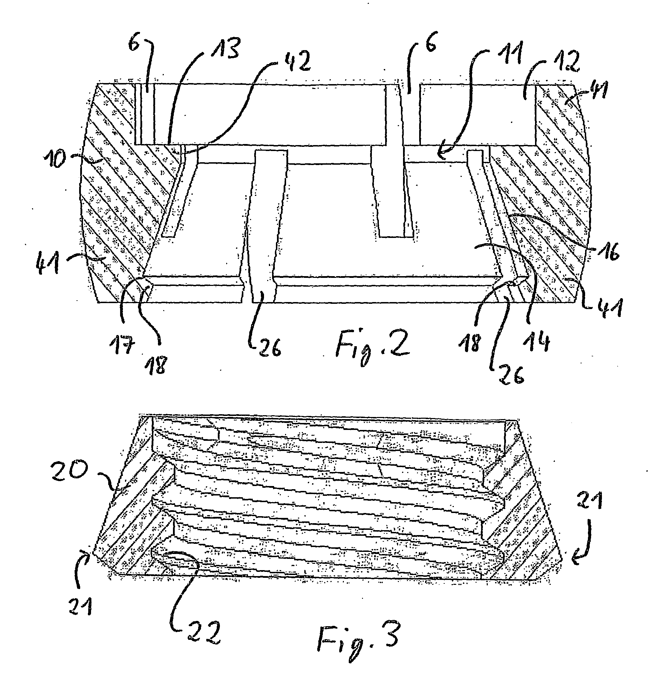

Das erste Einsatzelement 10 ist hier an vier Stellen im Abstand von 90 Grad wechselseitig von oben und unten teilweise geschlitzt, was besser in der Detailzeichnung der Fig. 2 zu erkennen ist. Gleiche Merkmale sind in allen Zeichnungen mit den gleichen Bezugszeichen versehen. Die nachfolgenden im Zusammenhang mit der Fig. 1 beschriebenen Merkmale des ersten Einsatzelementes 10 können gleichfalls in der Detailzeichnung von diesem aus der Fig. 2 ersehen werden. Zwei Schlitze 6 sind von oben, zwei Schlitze 6 sind von unten in das erste Einsatzelement 10 eingebracht.

In der Fig. 1 liegt die Schnittebene in den zwei gegenüberliegenden oben offenen Schlitzen 6. Das erste Einsatzelement 10 verfügt entlang der Achse 3 über eine durchgehende Bohrung 11 für die Durchführung eines Fixationselementes 5, wobei sich die Durchführung auf der knochenfernen Seite in eine Fixationselementkopfaufnahme 12 verbreitert und somit eine Schulter 13 definiert, auf der sich der Fixationselementkopf abstützen kann. Die Schulter 13 spannt eine Ebene auf, die senkrecht zur Hauptachse 3 des ersten Einsatzelementes 10 verläuft.

Auf der knochennahen Seite verfügt das erste Einsatzelement 10 entlang der Achse 3 über einen ebenfalls verbreiterten Hohlraum 14 zur Aufnahme eines zweiten Einsatzelementes 20.

Der zur Aufnahme des zweiten Einsatzelementes 20 vorgesehene Hohlraum 14 des ersten Einsatzelementes 10 verbreitert sich selbst auch in Richtung der knochennah 4 vorgesehenen Endes und bildet eine konische Wandfläche 16. Anstelle eines sich öffnenden Konus kann auch eine andere vorteilhafterweise bezüglich der Achse 3 rotationssymmetrische Form gewählt werden.

Am knochennahen Ende der konischen Wandfläche 16 ist ein Knick (oder alternativ auch ein stetiger Übergang durch eine Rundung) 17 vorgesehen, mit dem der Hohlraum 14 des ersten Einsatzelementes 10 in einen sich schliessenden Konus mit einer Wandfläche 18 übergeht. Die Wandfläche 18 kann auch als Teil einer umlaufenden nach innen ragenden Nase angesehen werden. Dabei ist es für die Haltefunktion ausreichend, wenn nur Teilbereiche des Umfanges die besagte nach innen ragende Nase 18 aufweisen.

Das zweite Einsatzelement 20 ist im wesentlichen komplementär zum Hohlraum 14 des ersten Einsatzelementes 10 ausgestaltet, so dass es mit Spiel in dem Hohlraum 14 des ersten Einsatzelementes 10 aufnehmbar ist. Die Fig. 3 zeigt eine geschnittene 3D-Ansicht des zweiten Einsatzelementes aus der Fig. 1, in der der an seinem Umkreis grösste Abschnitt 21 des zweiten Einsatzelementes 20 besser zu erkennen ist, der im Bereich des Überganges 17 des ersten Einsatzelementes 10 zu liegen kommt. Durch die Wandfläche 18 des ersten Einsatzelementes 10 wird das zweite Einsatzelement 20 im ersten Einsatzelement 10 gehalten.

Das zweite Einsatzelement 20 ist nicht geschlitzt und vorzugsweise inkompressibel. Es verfügt über eine durchgehende innengewindete Bohrung 22, in die das besagte Fixationselement 5 einschraubbar ist.

Die Funktionsweise der Vorrichtung ist wie folgt. Die auf dem Knochenbereich 4 aufgelegte Platte 1 ist in den Löchern 2, in denen die polyaxiale Fixierung vorgesehen ist, mit den eingesetzten beiden Einsatzelementen 10 und 20 vorbereitet. Das Fixationselement 5, beispielsweise eine Schraube, wird direkt oder über eine sie haltende Führung aufgesetzt und ausgerichtet, so dass die Hauptachse 3 der Schraube 5 mit der Hauptachse der beiden Einsatzelemente 10 und 20 übereinstimmt. Durch die kugelige Aussenform des ersten Einsatzelementes 10 ist ein breiter Winkelbereich auswählbar. Die Schraube 5 greift beim Eindrehen zuerst mit ihrem später in dem Knochen verankerten Aussengewinde 25 in das Innengewinde 22 des zweiten Einsatzelementes 20 ein und anschliessend in das Knochenmaterial 4. Dadurch bewegt sich der Schraubenkopf von der knochenfernen Seite auf die Platte 1 und das erste Einsatzelement 10 zu. Der Durchmesser der Knochenschraube 5 und die zugehörige Gewindegrösse können aber auch so gewählt sein, dass das besagte erste Aussengewinde 25 der Knochenschraube 5 ohne Berührung des ersten Einsatzelementes 10 durch dieses hindurchgeht. Nach einem zylindrischen Übergangsschaftbereich 27 verfügt die Schraube über ein zweites Aussengewinde 28. Das zweite Aussengewinde 28 ist zylindrisch und greift in das Gewinde 22 des zweiten Einsatzelementes 20 ein.

Neben der richtungsweisenden Führung der Schraube 5 in dem zweiten Einsatzelement 20 geschieht eine weitere Festlegung der Ausrichtung und Orientierung in dem Moment, in dem der Kopf des Fixationselementes 5 in den Hohlraum 12 eintritt, da dann Seitenwände des ersten Einsatzelementes 10 die vorher festgelegte Ausrichtung auch im ersten Einsatzelement 10 bestätigen, sofern der Durchmesser des Schaftes und der Gewinde 27 und 28 der Knochenschraube 5 entsprechend gross sind. Dann kommt zeitlich der Moment, zu dem das Fixationselement 5 soweit eingeschraubt ist, dass der Kopf auf der Schulter 13 aufliegt. Ab diesem Zeitpunkt bewirkt ein weiteres Drehen des Fixationselementes 5 eine axiale Bewegung des zweiten Einsatzelementes 20 entlang der Achse 3 auf den Hohlraum 14 zu. Dadurch bewegen sich die konischen Flächen 16 der beiden Einsatzelemente aufeinander zu und das innere nicht flexible zweite Einsatzelement 20 spreizt das flexible erste Einsatzelement 10 auf, so dass die kugeligen Aussenflächen desselben zumindest teilweise gegen die Wand 9 drücken. Somit ist es möglich, durch einfaches Festziehen des Fixationselementes 5 die voreingestellte polyaxiale Orientierung festzulegen und zu bestätigen.

Vorteilhafterweise sind die Platte 1 und die Einsatzelemente 10 und 20 aus medizinischem Stahl oder Titan oder einem anderen in der Medizintechnik verwendeten Werkstoff. Insbesondere kann als Material PEEK verwendet werden, was - neben anderen Materialien - zudem eine andere vorteilhafte Ausgestaltung des ersten Einsatzelementes 10 ermöglicht. Dann kann dieses erste Einsatzelement 10 ohne Schlitze ausgestaltet sein, da es durch die Wahl des Materials in sich selber flexibel ist. Ein solches Einsatzelement 10 ist dann vorzugsweise kohlefaserverstärkt. Wesentlich ist, dass das erste Einsatzelement elastisch verformbar ist, wobei die Art und Anzahl von Schlitzen und/oder die Materialwahl nur zwei Beispiele von möglichen Ausgestaltungen sind. Die besagte Verformbarkeit oder Flexibilität muss ein Aufspreizen des ersten Einsatzelementes 10 in radialer Richtung in Bezug auf die Aufnahme 9 ermöglichen. Im dargestellten Ausführungsbeispiel ist die Aufnahme 9 eine in der Dicke mittig der Platte 1 zentrierte Hohlkugel.

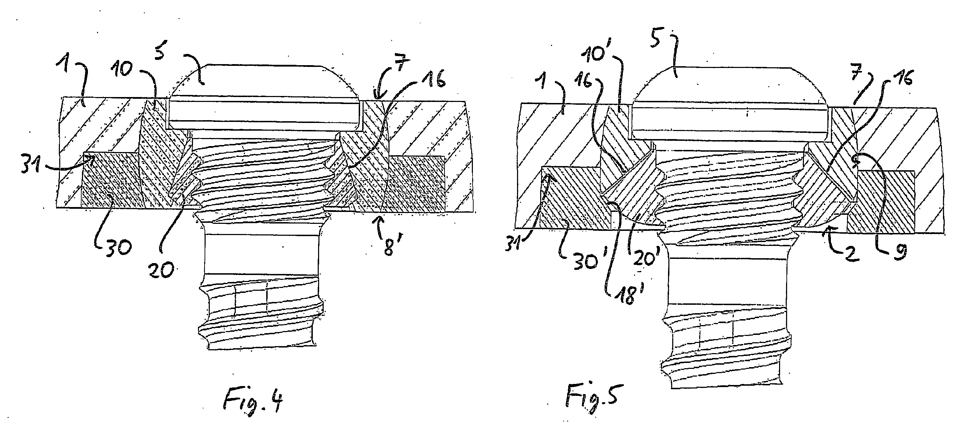

Die Fig. 4 und 5 zeigen geschnittene Seitenansichten von zwei abgewandelten Formen des ersten Ausführungsbeispiels der Vorrichtung. Der Bereich der ersten und zweiten Einsatzelemente 10 und 20 ist gleich ausgestaltet zu der in den Fig. 1 bis 3 dargestellten Ausführungsform. Wie oben erwähnt ist es wesentlich, dass der Einsatz in der Aufnahme 9 gehalten wird, was durch die hier in der Fig. 1 umlaufenden Vorsprüngen 7 bzw. 8 gewährleistet wird. Diese sind in der Fig. 1 die den Oberflächen der Platte 1 nahen Ränder der Aufnahme 9, die durch deren Hohlkugelform einen kleineren Durchmesser als die Tiefe der Aufnahme9 selber haben. Dies erfordert einen Kraftaufwand beim Einsetzen des Einsatzes.

Die Fig. 4 zeigt eine Ausgestaltung, bei der im knochennahen Bereich die Platte 1 eine ringförmige Ausnehmung 31 aufweist. Die Einsatzelemente 10 und 20 werden dann in der Aufnahme 9 zuerst eingesetzt und dadurch festgelegt, dass ein drittes Einsatzelement 30 nachfolgend als Ring in die entsprechende Ausnehmung 31 in der Platte 1 eingesetzt wird. Dabei kann die Fixierung durch Verpressung, Verschraubung, Verschweissung oder ein anderes medizintechnisch übliches Verfahren vorgesehen sein. Dieses dritte Einsatzelement 30 verfügt dann über eine kugelige Innenfläche, die die Aufnahme 9 der Platte 1 stetig ergänzt, so dass sich das erste Einsatzelement nun auf der knochennahen Seite vor der Implantation an der umlaufenden verjüngten Kante 8' abstützen kann. Die Implantation selber erfolgt in analoger Weise wie beim ersten Ausführungsbeispiel, das heisst durch Interaktion der beiden ersten und zweiten Einsatzelemente 10' und 20' über die komplementären Flächen 16, ohne dass hier das dritte Einsatzelement 30 eine wesentliche Funktion ausübt. Es nimmt lediglich die in der knochennahen Hälfte wirkenden Kräfte beim Aufspreizen des ersten Einsatzelementes 10 auf.

Für die Beschreibung zum ersten Einsatzelement 10' nach der zweiten Abwandlung des ersten Ausführungsbeispiels wird auf die Beschreibung zur Fig. 1 verwiesen. Der Unterschied zwischen den beiden ersten Einsatzelementen 10 und 10' besteht darin, dass das erste Einsatzelement 10' nach dieser Abwandlung des Ausführungsbeispiels keinen Bereich 17 hat, der einen Knick oder auch einen stetigen Übergang durch eine Rundung von einem sich erweiternden Abschnitt in einen sich verjüngenden Abschnitt aufweist. Vielmehr endet die konische Wandfläche 16 oder geht beispielsweise in eine Fläche parallel zur Hauptebene der Platte 1 über. Das zweite Einsatzelement 20' weist dagegen einen Bereich grössten Durchmesser auf. Dadurch sind das erste Einsatzelement 10' und das zweite Einsatzelement 20' von der unteren Seite in die Öffnung 2 der Platte 1 frei einsetzbar. Sie werden in der Aufnahme 9 dadurch festgelegt, dass ein drittes Einsatzelement 30' vorgesehen ist, welches als Ring in eine entsprechende Ausnehmung 31 in der Platte 1 eingesetzt wird. Dabei kann die Fixierung durch Verpressung, Verschraubung, Verschweissung oder ein anderes medizintechnisch übliches Verfahren vorgesehen sein. Dieses dritte Einsatzelement 30' verfügt dann über eine konische, sich zum Knochen 4 hin verjüngende Fläche 18', gegen die sich das zweite Einsatzelement 20' vor der Implantation abstützen kann. Die Implantation selber erfolgt in analoger Weise wie bei den oben erwähnten Aisführungen des ersten Ausführungsbeispiels, das heisst durch Interaktion der beiden ersten und zweiten Einsatzelemente 10' und 20' über die komplementären Flächen 16.

Anstelle dieser in den Zeichnungen als konische Flächen dargestellte komplementären Flächen 16 können auch andere einen Kraft- und/oder Reibschluss ermöglichende Flächenformen vorgegeben werden, die sich gegeneinander verspannen können, wenn ein Fixationselement durch die Einsatzelemente 10' und 20' vorgeschoben wird.

Dabei werden die beiden Einsatzelemente 10 und 20 bzw. 10' und 20' jeweils durch Aufspreizen der Seitenbereiche 41 der ersten Einsatzelemente 10, 10' gegen die kugeligen Innenwände der Platte 1 festgesetzt, was durch das axiale Festsetzen der Unterseite des Fixationselementkopfes auf der Schulter 13 und gegen den zentralen Bereich 42 des ersten Einsatzelementes 10, 10' geschieht. Dieses Festsetzen erzeugt die axiale Gegenbewegung des zweiten Einsatzelementes 20 bzw. 20' in Richtung seiner Längsachse 3, welches dadurch die Flügelbereiche 41 spreizt, deren Innenflächen 16 in einem sich entsprechend zum zentralen Bereich 42 verjüngenden komplementären Eingriff mit den entsprechenden Flächen des zweiten Einsatzelementes 20 oder 20' stehen.

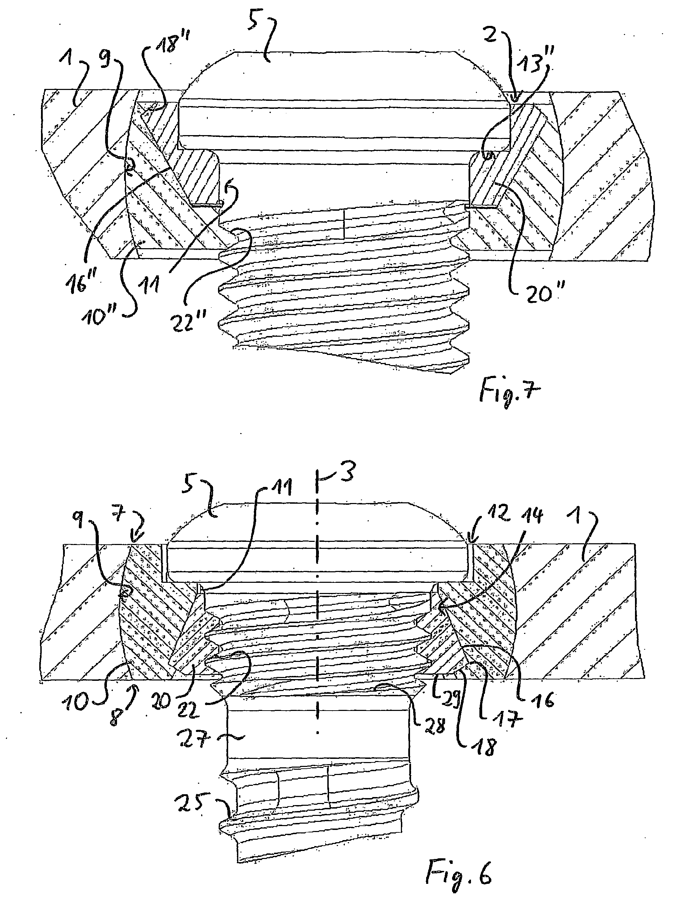

Die Fig. 6 zeigt eine in einer anderen Ebene des ersten Einsatzelementes 10 geschnittene Seitenansicht des ersten Ausführungsbeispiels nach Fig. 1, wobei der Schnitt durch das Vollmaterial des ersten Einsatzelementes 10 geht und somit keine Schlitze erkennbar sind. Somit entspricht diese Fig. auch einem Ausführungsbeispiel mit einem in sich selber flexiblen ersten Einsatzelement, z.B. aus PEEK.

Alternativ zu dem Gewinde 22 kann das zweite Einsatzelement 20 auch mit einer glatten Innenbohrung versehen sein, so dass kein direkter Kontakt zwischen Schraube 5 und zweitem Einsatzelement 20 besteht. Dann kann die Funktion der axialen Bewegung des zweiten Einsatzelementes 20 durch die entsprechend weiter in Knochennähe geführte Unterseite 29 des zweiten Einsatzelementes 20 realisiert werden, welches beim Aufsetzen auf die Unterlage gegen das erste Einsatzelement 10 drückt und dieses verspreizt.

Die Fig. 7 schliesslich zeigt eine geschnittene Seitenansicht eines zweiten Ausführungsbeispiels der Vorrichtung. In den Figuren auftretenden gleiche Merkmale sind jeweils mit den gleichen Bezugszeichen versehen worden. Hier ist die Schulter 13" zum Abstützen im inneren Einsatzelement vorgesehen, welches durch den Kopf der Schraube 5 gegen das äussere Einsatzelement gedrückt wird (und dieses aufspreizt). Damit das gelingt, ist im äusseren Einsatzelement ein Innengewinde 22" vorgesehen. Die Ausgestaltung der Nase 18" und der konischen Eingriffsflächen 16" kann analog zu dem ersten Ausführungsbeispiel ausgeführt sein.

## Patentansprüche

1. Implantierbare orthopädische Vorrichtung mit einem Lastträgerelement (1), mit mindestens einem polyaxial orientierbaren Fixationselement, wobei in dem Lastträgerelement (1) mindestens eine Öffnung (2) für den Durchgang von polyaxial orientierbaren Fixationselementen (5) vorgesehen ist, und mit einem Einsatz (10, 20; 10', 20'; 10", 20"), der in die besagte Öffnung (2) in einer Aufnahme (9) einsetzbar ist, wobei der Einsatz (10, 20; 10', 20'; 10", 20") eine zu der inneren Form der Aufnahme (9) komplementäre äussere Form aufweist, die eine polyaxiale Rotation des Einsatzes in der Aufnahme (9) gestattet, wobei der Einsatz (10, 20; 10', 20'; 10", 20") eine zentrale Durchbohrung (11, 22; 22") zur Aufnahme des Körpers des Fixationselementes (5) aufweist und wobei die besagte implantierbare orthopädische Vorrichtung Mittel (18; 18', 30'; 18") zum Halten des Einsatzes (10, 20; 10', 20'; 10", 20") in der Aufnahme (9) aufweist, **dadurch gekennzeichnet, dass** der Einsatz aus zwei Einsatzelementen (10, 20; 10', 20'; 10", 20") mit der besagten Durchbohrung (11, 22; 22") besteht, dass das erste Einsatzelement (10; 10'; 10") die besagte äussere Form aufweist, die die polyaxiale Rotation desselben in der Aufnahme (9) gestattet, dass das erste Einsatzelement (10; 10'; 10") einen zentralen inneren Hohlraum (14) aufweist, in dem das zweite Einsatzelement (20, 20', 20") einsetzbar ist, und dass das erste Einsatzelement (10, 10'; 10") durch eine axiale (3) Verschiebung der beiden Einsatzelemente (10 zu 20; 10' zu 20'; 10" zu 20") gegeneinander in dem Lastträgerelement (1) mindestens teilweise aufspreizbar ist, um Position und Ausrichtung des Fixationselementes (5) in der Vorrichtung polyaxial einstellbar zu blockieren.

2. Vorrichtung nach Anspruch 1, **dadurch gekennzeichnet, dass** das erste Einsatzelement (10, 10', 10") mindestens einen Schlitz (6) derart aufweist, dass bei der axialen (3) Verschiebung der beiden Einsatzelemente (10 zu 20; 10' zu 20'; 10" zu 20") gegeneinander das besagte erste Einsatzelement (10; 10'; 10") in dem Lastträgerelement (1) teilweise aufspreizbar ist.

3. Vorrichtung nach Anspruch 2, **dadurch gekennzeichnet, dass** das erste Einsatzelement (10, 10', 10") in radialer Richtung bezüglich der Aufnahme (9) flexibel derart ist, dass bei der axialen (3) Verschiebung der beiden Einsatzelemente (10 zu 20; 10' zu 20'; 10" zu 20") gegeneinander das besagte erste Einsatzelement (10; 10'; 10") in dem Lastträgerelement (1) teilweise aufspreizbar ist.

4. Vorrichtung nach Anspruch 1 oder 2, **dadurch gekennzeichnet; dass** das erste Einsatzelement (10, 10') knochenfern eine um die zentrale Durchbohrung (11) angeordnete Schulter (13) aufweist, die als Anschlag für den Kopf des einzusetzenden Fixationselementes vorgesehen und über einen mittleren Verbindungsabschnitt (42) des ersten Einsatzelementes (10, 10') gegenüber dem Hohlraum (14) angeordnet ist.

5. Vorrichtung nach Anspruch 3, **dadurch gekennzeichnet, dass** die Durchbohrung (22) des zweiten Einsatzelementes (20, 20') mindestens teilweise ein Innengewinde zum Eingriff in ein Aussengewinde des einzusetzenden Fixationselementes (5) umfasst.

6. Vorrichtung nach einem der Ansprüche 1 bis 4, **dadurch gekennzeichnet, dass** sich der zentrale Hohlraum (14) in Richtung des Kopfes des einzusetzenden Fixationselementes (5) verjüngt und dass das zweite Einsatzelement (20, 20') mit einer im wesentlichen komplementär zu dem zentralen Hohlraum (14) verjüngten Form ausgestaltet ist.

7. Vorrichtung nach einem der Ansprüche 1 oder 2, **dadurch gekennzeichnet, dass** das zweite Einsatzelement (20") knochenfern eine um die zentrale Durchbohrung (11) angeordnete Schulter (13") aufweist, die als Anschlag für den Kopf des einzusetzenden Fixationselementes (5) vorgesehen ist, und dass die zentrale Durchbohrung des ersten Einsatzelementes (10") mindestens teilweise ein Innengewinde (22") zum Eingriff in ein Aussengewinde des einzusetzenden Fixationselementes (5) umfasst.

8. Vorrichtung nach Anspruch 6, **dadurch gekennzeichnet, dass** sich der zentrale Hohlraum (14) in Richtung des Kopfes des einzusetzenden Fixationselementes (5) verbreitert und dass das zweite Einsatzelement (20") mit einer im wesentlichen komplementär zu dem zentralen Hohlraum (14) verjüngten Form ausgestaltet ist.

9. Vorrichtung nach einem der vorstehenden Ansprüche, **dadurch gekennzeichnet, dass** die Mittel (18', 30') zum Halten des Einsatzes (10', 20') in der Aufnahme (9) ein drittes von Knochenseite in die Platte (1) einsetzbares ringförmiges Einsatzelement (30') umfassen, welches eine nach innen weisende und in Eingriff mit dem zweiten Einsatzelement (20') bringbare Schulter (18') aufweist.

10. Vorrichtung nach einem der vorstehenden Ansprüche 1 bis 7, **dadurch gekennzeichnet, dass** die Mittel (18) zum Halten des Einsatzes (10, 20) in der Aufnahme (9) eine zumindest zum Teil umlaufende Nase (18) am knochennahen Ende des ersten Einsatzelementes (10) umfassen.

## Claims

1. Implantable orthopedic device with a load-bearing element (1), with at least one polyaxially-orientable fixation element, wherein in the load-bearing element (1) at least one opening (2) for the passage of polyaxially-orientable fixation elements (5) is provided, and with an insert (10, 20; 10', 20'; 10", 20"), which may be inserted in the said opening (2) into a mounting (9), wherein the insert (10, 20; 10', 20'; 10", 20"), exhibits an outer shape that is complementary to the inner shape of the mounting (9), allowing a polyaxial rotation of the insert in the mounting (9), wherein the insert (10, 20; 10', 20'; 10", 20") exhibits a central transfixion (11, 22; 22") for accepting the body of the fixation element (5) and wherein the said implantable orthopedic device exhibits means (18; 18', 30; 18") for holding the insert (10, 20; 10', 20'; 10", 20") in the mounting (9), **characterized in that** the insert consists of two insert elements (10, 20; 10', 20'; 10", 20") with the said transfixion (11, 22; 22"), **in that** the first insert element (10; 10';10") exhibits the said outer form, which allows the polyaxial rotation thereof in the mounting (9), **in that** the first insert element (10; 10'; 10") exhibits a central inner hollow space (14), in which the second insert element (20, 20', 20") may be inserted, and **in that** the first insert element (10, 10'; 10") is at least partially spreadable by axial (3) displacement of the two insert elements (10 to 20; 10' to 20'; 10" to 20") against each other in the load-bearing element (1), in order to block the position and orientation of the fixation element (5) in a polyaxially-orientable manner, when inserted in the device.

2. Device according to claim 1, wherein the first insert element (10, 10', 10") exhibits at least one slit (6) of such type that with the axial (3) displacement of the two insert elements (10 to 20; 10' to 20'; 10" to 20") against each other, the said first insert element (10, 10', 10") can be partially spread apart in the load-bearing element (1).

3. Device according to claim 2, wherein the first insert element (10, 10', 10") is flexible in radial direction with respect to the mounting (9) in such a way that with the axial (3) displacement of the two insert elements (10 to 20; 10' to 20'; 10" to 20") against each other, the said first insert element (10; 10'; 10") can be partially spread apart in the load-bearing element (1).

4. Device according to claim 1 or 2, wherein the first insert element (10, 10') on the side away from the bone exhibits a shoulder (13) around the central transfixion (11), which is provided as a stop for the head of the fixation element to be inserted and is arranged over a central connecting section (42) of the first insert element (10, 10') opposite the hollow space (14).

5. Device according to claim 3, wherein the transfixion (22) of the second insert element (20, 20') comprises at least partially an inner threading for engaging in the outer threading of the fixation element (5) to be inserted.

6. Device according to any of claims 1 to 4, wherein the central hollow space (14) tapers in direction of the head of the fixation element (5) to be inserted and wherein the second insert element (20, 20') is designed with a tapering shape that is essentially complementary to the central hollow space (14).

7. Device according to claim 1 or 2, wherein the second insert element (20") on the side away from the bone exhibits a shoulder (13") around the central transfixion (11), which is provided as a stop for the head of the fixation element (5) to be inserted, and that the central transfixion of the first insert element (10") comprises at least partially an inner threading (22") for engaging in the outer threading of the fixation element (5) to be inserted.

8. Device according to claim 6, wherein the central hollow space (14) widens in direction of the head of the fixation element (5) to be inserted and wherein the second insert element (20") is designed with a tapered shape that is essentially complementary to the central hollow space (14).

9. Device according to any of the preceding claims, wherein the means (18', 30') for holding the insert (10', 20') in the mounting (9) comprise a third ring-shaped insert element (30') that may be inserted in the plate (1) from the bone side, which exhibits an inward-directed shoulder (18') that can be brought to engage the second insert element (20').

10. Device according to any of the claims 1 to 7, wherein the means (18) for holding the insert (10, 20) in the mounting (9) comprise an at least partially circumferential nose (18) at the end of the first insert element (10) near the bone.

## Revendications

1. Dispositif orthopédique implantable avec un élément de support avec au moins un élément de fixation polyaxial orientable, où au moins un alésage (2) est prévu pour le passage des éléments de fixation (5) polyaxiaux orientables, et avec un insert (10, 20; 10', 20' ; 10", 20"), qui peut être inséré dans ledit alésage (2) d'une réception (9), où l'insert (10, 20; 10', 20' ; 10", 20") possède une forme extérieure complémentaire à la forme intérieure de la réception (9), qui permet une rotation polyaxiale de l'insert dans la réception (9), où l'insert (10, 20; 10', 20' ; 10", 20") comprend un alésage central (11, 22 ; 22") pour la réception du corps de l'élément de fixation (5) et où le dispositif orthopédique implantable comprend des moyens (18, 18', 30' ; 18") pour tenir l'insert (10, 20; 10', 20' ; 10", 20") dans la réception (9), **caractérisé en ce que** l'insert comprend deux éléments d'insert (10, 20; 10', 20'; 10", 20") avec ledit alésage (11, 22 ; 22"), **en ce que** le premier élément d'insert (10 ; 10' ; 10") possède ladite forme extérieure, qui permet la rotation polyaxiale dans la réception (9), **en ce que** le premier élément d'insert (10 ; 10' ; 10") possède une cavité intérieure centrale (14), dans laquelle le deuxième élément d'insert (20, 20', 20") peut être inséré, et **en ce que** le premier élément d'insert (10, 10' ; 10") peut être au moins partiellement écarté par un déplacement axial (3) des deux éléments d'insert (10 à 20 ; 10' à 20' ; 10" à 20") l'un vers l'autre dans l'élément de support (1) pour bloquer, dans le dispositif et d'une manière polyaxiale, la position et l'orientation des éléments de fixation (5).

2. Dispositif selon la revendication 1, **caractérisé en ce que** le premier élément d'insert (10, 10', 10") comprend au moins une fente (6), tel ce que ledit premier élément d'insert (10, 10', 10") peut être au moins partiellement écarté dans l'élément du support (1) lors du déplacement axial (3) des deux éléments d'insert (10 à 20 ; 10' à 20' ; 10" à 20") l'un vers l'autre.

3. Dispositif selon la revendication 2, **caractérisé en ce que** le premier élément d'insert (10, 10', 10") est flexible dans sa direction radiale par rapport à la réception (9), tel que ledit premier élément d'insert (10, 10', 10") peut être au moins partiellement écarté dans l'élément du support (1) lors du déplacement axial (3) des deux éléments d'insert (10 à 20 ; 10' à 20' ; 10" à 20") l'un vers l'autre.

4. Dispositif selon la revendication 1 ou 2, **caractérisé en ce que** le premier élément d'insert (10, 10') comprend une épaule (13) disposée autour de l'alésage central (11) loin de l'os, qui est prévue en tant que butoir pour la tête de l'élément de fixation à être inséré et est arrangée au-dessus de la section de raccordement (42) centrale du premier élément d'insert (10, 10') par rapport à la cavité (14).

5. Dispositif selon la revendication 3, **caractérisé en ce que** le percement (22) du deuxième élément d'insert (20, 20') comprend au moins partiellement un filetage intérieur pour engager un filetage extérieur de l'élément de fixation (5) à être inséré.

6. Dispositif selon l'une des revendications 1 à 4, **caractérisé en ce que** la cavité centrale (14) se rétrécit dans la direction de la tête de l'élément de fixation (5) à être inséré et **en ce que** le deuxième élément d'insert (20, 20') est arrangé avec une forme rétrécissante qui est essentiellement complémentaire à la cavité centrale (14).

7. Dispositif selon l'une des revendications 1 ou 2, **caractérisé en ce que** le deuxième élément d'insert (20") comprend une épaule (13") disposée autour de l'alésage central (11) loin de l'os, qui est prévue en tant que butoir pour la tête de l'élément de fixation (5) à être inséré et **en ce que** le percement central du premier élément d'insert (10") comprend au moins partiellement un filetage intérieur (22") pour engager un filetage extérieur de l'élément de fixation (5) à être inséré.

8. Dispositif selon la revendication 6, **caractérisé en ce que** la cavité centrale (14) s'élargit dans la direction de la tête de l'élément de fixation (5) à être inséré et **en ce que** le deuxième élément d'insert (20") est arrangé avec une forme rétrécissante qui est essentiellement complémentaire à la cavité centrale (14).

9. Dispositif selon l'une des revendication précédentes, **caractérisé en ce que** les moyens (18', 30') pour tenir l'insert (10', 20') dans la réception (9) comprennent un troisième élément d'insert (30') annulaire qui peut être introduit du coté de la face de l'os dans l'élément du support (1), qui comprend une épaule (18') dirigée vers l'intérieur qui peut être amenée à engager le deuxième élément d'insert (18').

10. Dispositif selon l'une des revendications 1 à 7, **caractérisé en ce que** les moyens (18) pour tenir l'insert (10, 20) dans la réception (9) comprennent un nez (18) qui est au moins partiellement circonférentiel à l'extrémité près de l'os du premier élément d'insert (10).
